# EUROPEAN PATENT APPLICATION

(11) **EP 3 210 643 A1**
(43) Date of publication of application: **30.08.2017**
(21) Application number: 16156937.1
(22) Date of filing: 23.02.2016
(51) Int. Cl.: A61M 16/04, A61M 25/02

(54) **ENDOTRACHEAL TUBE HOLDER**

(71) Applicant: Besmed Health Business Corp., New Taipei City 248 (TW)
(72) Inventor: HSIUNG, Tao-Tsun, New Taipei City (TW)
(74) Representative: Zacco GmbH

(57) **Abstract**

An endotracheal tube holder (1) comprises a holder body (10), a cushion (20) and a pushing component (30). The holder body (10) comprises an inner surface (11), an aperture (13), a bite wall (14) and a receiving portion (15). The receiving portion (15) comprises two parallel side walls (151) connected to the inner surface (11), a receiving space (153) formed between the two side walls (151), an opening (155) located at a side of the receiving portion (15), and two protruding portions (157) respectively located at the two side walls (151). The opening (155), the receiving space (153), and the aperture (13) communicate to each other. The cushion (20) is located at the inner surface (11). The pushing component (30) is mounted through the opening (155) and comprises two blocks (333) selectively buckled to the two protruding portions (157) that can be quickly fixed on the endotracheal tube (50).

## Description

### 1. Field of the Invention

The present invention relates to an endotracheal tube holder, especially to the endotracheal tube holder which can be quickly mounted and secured.

### 2. Description of the Prior Arts

Medical care situations in need of intubation can be divided into three situations: first, resuscitation, if a patient is still unable to breathe on his/her own after cardiopulmonary resuscitation (CPR) for a certain duration, the endotracheal intubation and advanced life support will be applied; second, major surgery on the head or chest, for which endotracheal intubation should be performed since the major surgery requires general anesthesia rather than local anesthesia or partial anesthesia; the third, respiratory failure caused by pneumonia, for which endotracheal intubation is needed in order to provide sufficient oxygen for the patient.

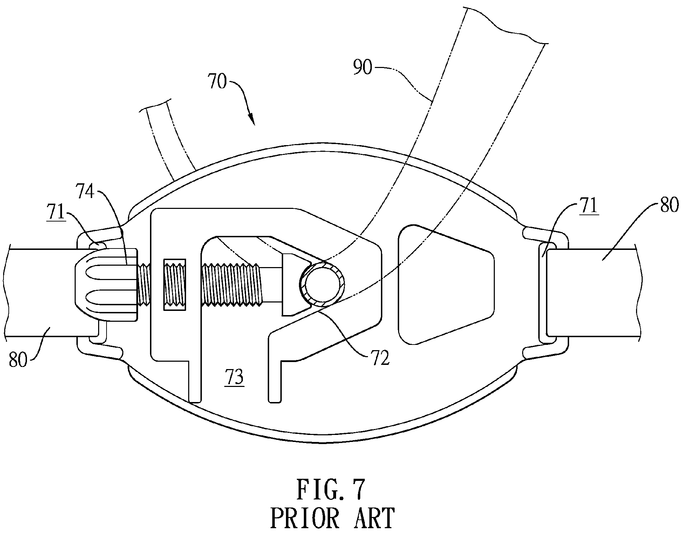

As shown in Fig. 7, the conventional endotracheal tube holder 70 is used for fixing an endotracheal tube 90 during endotracheal intubation. When the conventional endotracheal tube holder 70 is in use, a fixing belt 80 is inserted into two openings 71 located at two ends of the conventional endotracheal tube holder 70, and the conventional endotracheal tube holder 70 is fixed at the patient's head by the fixing belt 80. Then, the endotracheal tube 90 is abutted against a bite wall 72 and is inserted into the patient's mouth through the aperture 73. The endotracheal tube 90 is fixed by screwing an end of the screw 74.

However, the endotracheal tube 90 is fixed at the bite wall 72 by the screw 74 of the conventional endotracheal tube holder 70, so that the thread density of the screw 74 need to be high in order to avoid loosening of the screw 74 causing discomfort of the patient due to moving or vibrating of the endotracheal tube 90. When the screw thread density is high, the time for securing the endotracheal tube 90 is increased because the inward displacement for one round of rotation of the screw 74 is small, such that the patient will suffer pain much longer due to the insertion and securing of the endotracheal tube 90. The vibration occurs during screwing of the screw 74, which further increases the patient's pain. Beside, the opening 71 of the conventional endotracheal tube holder 70 is formed in an elongated shape, so the fixing belt 80 inserted into the opening 71 is easy to slide and fall off, such that the patient feels uncomfortable due to the endotracheal tube 90 moving or vibrating. Therefore, the disadvantages in prior arts should be resolved.

According to the above description, the objective of the present invention is to improve the structure of the conventional endotracheal tube holder to reduce the time for inserting and securing the endotracheal tube so that the patient's discomfort can be reduced; another objective of the present invention is to decrease the movement or vibration generated by inserting the endotracheal tube, thereby decreasing the patient's discomfort.

To achieve the above purpose of the present invention, the objective of the invention is to provide a holder body, a cushion and a pushing component. The holder body comprises an inner surface, an aperture, a bite wall, a receiving portion, and two fixed portions. The aperture is formed through the inner surface and located at the middle of the holder body. The bite wall is formed on and extends from the inner surface. The receiving portion comprises two side walls, a receiving space, an opening, and two protruding portions. The side walls are parallel to each other and are connected to the inner surface. The receiving space is formed between the two side walls. The opening is located at a side of the receiving portion opposite to the bite wall; the opening and the receiving space communicate with the aperture. The two protruding portions are respectively located at the two side walls. The two fixed portions are respectively located at two ends of the holder body and connected to the inner surface. The cushion is located at the inner surface of the holder body and the bite wall. The pushing component is mounted through the opening, and the pushing component comprises two middle side walls and two blocks. The two middle side walls are parallel to each other; the two blocks are respectively located at the two middle side walls, and the two blocks are selectively buckled on the two protruding portions.

Preferably, the receiving portion further comprises an abutment plate and two grooves. The abutment plate is connected to a side of the two side walls opposite to the inner surface; the two grooves are respectively located at the abutment plate and extend to the aperture, and the two grooves are respectively parallel to the two side walls.

More preferably, the pushing component comprises a front segment, a middle segment, and a pressing segment. The front segment has an abutment surface and two front side walls, two ends of the abutment surface are respectively connected to the two front side walls, and the two front side walls are parallel to each other. The middle segment comprises a bottom wall, the two middle side walls and the two blocks; the bottom wall is located at an end of the front segment that is opposite to the abutment surface, and the two middle side walls are respectively connected to two sides of the bottom wall. The pressing segment is located at an end of the bottom wall opposite to the front segment.

More preferably, the middle segment further comprises two side grooves respectively located at the connection site between the two middle side walls and the bottom wall.

More preferably, the pushing component further comprises a first cushion segment located between the front segment and the middle segment.

More preferably, the pushing component further comprises a second cushion segment located between the middle segment and the pressing segment.

More preferably, the receiving portion further comprises at least one guiding groove formed on and extending from the opening and connected to the abutment plate; wherein the front segment further comprises at least one front guiding strip located at one of the two front side walls, and the at least one front guiding strip is slid into the at least one guiding groove.

More preferably, the receiving portion further comprises at least one guiding groove formed on and extending from the opening and connected to the abutment plate; wherein the pressing segment comprises a rear bottom wall, two rear side walls and at least one rear guiding strip. An end of the rear bottom wall is connected to the bottom wall. The two rear side walls are respectively connected to two sides of the rear bottom wall, and the two rear side walls are parallel to each other. The at least one rear guiding strip is located at one of the two rear side walls, and the at least one rear guiding strip is slid into the at least one guiding groove.

Preferably, each protruding portion comprises multiple protrusions, and each one of the protrusions has a first inclined plane toward the opening; wherein each block has a second inclined plane toward the bite wall.

Preferably, each fixed portion has a wavy opening.

The advantages of the endotracheal tube holder of the present invention is that the pushing component is pushed and coordinated with the fixed structure of the two blocks and the two protruding portions, and the endotracheal tube can be quickly fixed so that the time for inserting and securing the endotracheal tube is reduced, thereby reducing the patient's discomfort.

Other objectives, advantages and novel features of the invention will become more apparent from the following detailed description when taken in conjunction with the accompanying drawings.

### IN THE DRAWINGS

Fig. 1 is an exploded view of an endotracheal tube holder of the present invention.
Fig. 2 is an exploded view of a holder body and a pushing component of the endotracheal tube holder of the present invention.
Fig. 3 illustrates an operational and front view of the endotracheal tube holder of the present invention.
Fig. 4 illustrates an operational and rear view of the endotracheal tube holder of the present invention.
Fig. 5 illustrates an operational and cross-sectional view of the endotracheal tube holder of the present invention.
Fig. 6 illustrates another operational and cross-sectional view of the endotracheal tube holder of the present invention.
Fig. 7 illustrates an operational view of the conventional endotracheal tube holder.

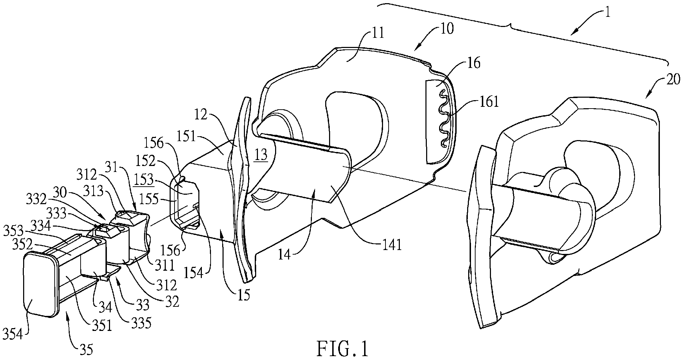

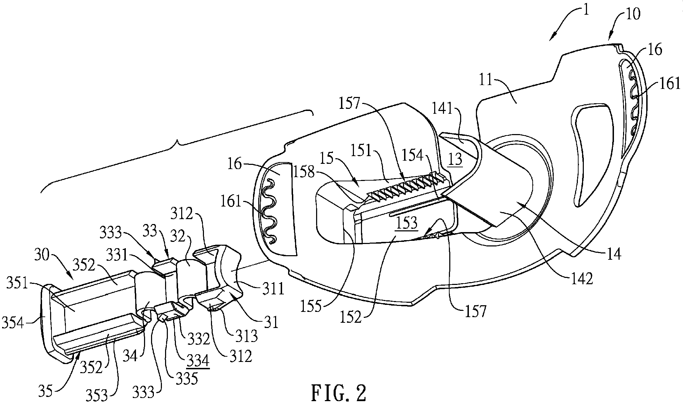

As shown in Fig. 1 and Fig. 2, the present invention provides an endotracheal tube holder 1, comprising a holder body 10, a cushion 20 and a pushing component 30.

The material of the holder body 10 is thermoplastic rubber and polypropylene, and the holder body 10 comprises an inner surface 11, an outer surface 12, an aperture 13, a bite wall 14, a receiving portion 15, and two fixed portions 16.

The inner surface 11 and the outer surface 12 are separately located at two sides of the holder body 10, and the inner surface 11 is opposite to the outer surface 12. The aperture 13 is located at the middle of the holder body 10, and more specifically, the aperture 13 is formed through the inner surface 11 and the outer surface 12, and is located at the middle of the holder body 10. The bite wall 14 is formed on and extending from the inner surface 11 and away from the outer surface 12, and the bite wall 14 has an inner wall 141 and an outer wall 142 opposite to the inner wall 141; the inner wall 141 of the bite wall 14 is toward the aperture 13. The receiving portion 15 is formed on and extending from the outer surface 12 to the inner surface 11.

The receiving portion 15 comprises two side walls 151, an abutment plate 152, a receiving space 153, two grooves 154, an opening 155, two guiding grooves 156, and two protruding portions 157.

As shown in Fig. 1 to Fig. 3, the two side walls 151 are parallel and have two sides, one side of the two side walls 151 is connected to the inner surface 11, the other side of the two side walls 151 is connected to the abutment plate 152, and the receiving space 153 is formed among the two side walls 151 and the abutment plate 152. The receiving space 153 communicates to the aperture 13. The two grooves 154 are located at the abutment plate 152 and extend to the aperture 13, and the two grooves 154 are respectively parallel to the two side walls 151 so that the abutment plate 152 can be moved toward the receiving space 153. The opening 155 is located at a side of the receiving portion 15 away from the aperture 13 and opposite to the bite wall 14, and the opening 155 communicates with the receiving space 153. The two guiding grooves 156 are respectively located at the two side walls 151 and extend to the opening 155. The two protruding portions 157 are respectively formed on the two side walls 151, and each one of the protruding portions 157 is between the opposite two guiding grooves 156 and the inner surface 11. Each one of the protruding portions 157 comprises multiple protrusions, each one of the protrusions has a first inclined plane 158 toward the opening 155, and each first inclined plane 158 is respectively toward its opposite side wall 151.

The two fixed portions 16 are respectively located at two ends of the holder body 10 and connected to the inner surface 11 and the outer surface 12. Each fixed portion 16 has a wavy opening 161 so that a fixing belt 40 could be crossed through and fixing the endotracheal tube holder 1 on the patient's head by the medical staff.

The material of the cushion 20 is thermoplastic rubber and polypropylene, and the cushion 20 is attached with the inner surface 11 of the holder body 10 and the outer wall 142 of the bite wall 14, thereby providing comfort in biting. The aperture 13 and the receiving portion 15 are exposed from the cushion 20 so the aperture 13 and the receiving space 153 can communicate with the external environment of the endotracheal tube holder 1. When the endotracheal tube holder 1 of the present invention is in use, a side of the cushion 20 opposite to the holder body 10 is toward the patient's head. In another preferred embodiment of the present invention, the cushion 20 and the holder body 10 are integrated.

The pushing component 30 is inserted into the opening 155, thereby moving along an axis parallel to the two side walls 151 in the receiving space 153. The pushing component 30 comprises a front segment 31, a first cushion segment 32, a middle segment 33, a second cushion segment 34, and a pressing segment 35.

The front segment 31 has an abutment surface 311, two front side walls 312 and two front guiding strips 313. The abutment surface 311 is formed in a concave arc shape to correspond to the shape of an endotracheal tube 50, and two ends of the abutment surface 311 are respectively connected to the two front side walls 312. The two front side walls 312 are parallel to each other. The two front guiding strips 313 are respectively located at the two front side walls 312, and the front guiding strips 313 are respectively slid into the guiding grooves 156.

The first cushion segment 32 is located at an end of the front segment 31 opposite to the abutment surface 311, and the cross-section of the first cushion segment 32 is in a U shape.

The middle segment 33 is located at an end of the first cushion segment 32 opposite to the front segment 31, the middle segment 33 comprises a bottom wall 331, two middle side walls 332, two blocks 333, and two side grooves 334. An end of the bottom wall 331 is connected to the first cushion segment 32. The two middle side walls 332 are respectively connected to two sides of the bottom wall 331, and the two middle side walls 332 are parallel to each other. More specifically, one of the two middle side walls 332 and one of the two front side walls 312 are at the same plane, the other one of the two middle side walls 332 and the other one of the two front side walls 312 are at the same plane. The two blocks 333 are respectively located at the two middle side walls 332, and each block 333 has a second inclined plane 335 toward the front segment 31. Each second inclined plane 335 of the two blocks 333 faces away from the opposite middle side wall 332, and the two blocks 333 are selectively buckled on the two protruding portions 157. The two side grooves 334 are respectively located at the connection site between the two middle side walls 332 and the bottom wall 331 so that the two middle side walls 332 can be moved toward each other.

The second cushion segment 34 is located at an end of the bottom wall 331 opposite to the first cushion segment 32, and the cross-section of the second cushion segment 34 is in a U shape.

The pressing segment 35 is located at an end of the second cushion segment 34 opposite to the middle segment 33, the pressing segment 35 has a rear bottom wall 351, two rear side walls 352, two rear guiding strips 353, and a pressing portion 354. An end of the rear bottom wall 351 is connected to the second cushion segment 34. The two rear side walls 352 are respectively connected to two sides of the rear bottom wall 351, and the two rear side walls 352 are parallel to each other. More specifically, one of the two rear side walls 352 and one of the two middle side walls 332 are at the same plane, and the other one of the two rear side walls 352 and the other one of the two middle side walls 332 are at the same plane. The two rear guiding strips 353 are respectively located at the two rear side walls 352, and the two rear guiding strips 353 are selectively slid into the two guiding grooves 156. The pressing portion 354 is located at an end of the rear bottom wall 351 opposite to the second cushion segment 34.

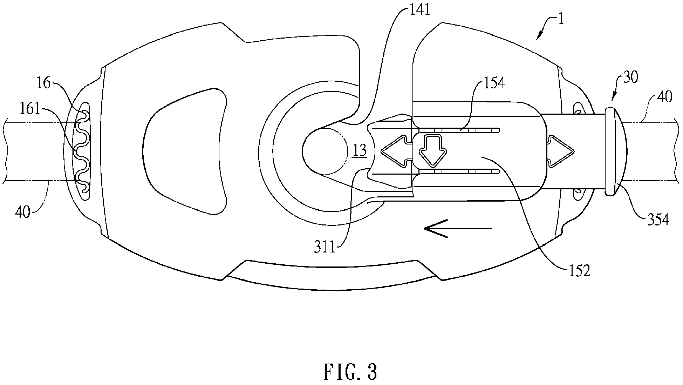

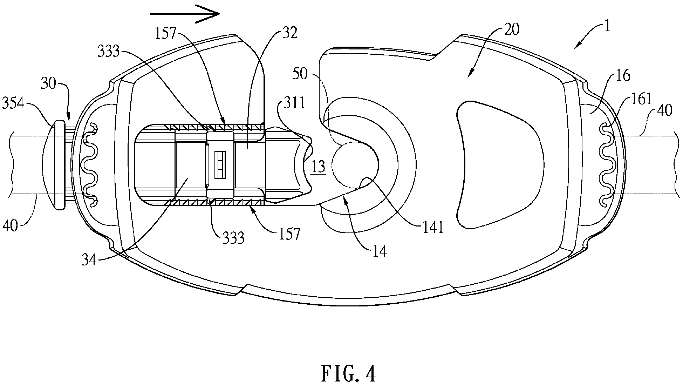

As shown in Fig. 3 and Fig. 4, when the endotracheal tube holder 1 of the present invention is in use, two ends of the fixing belt 40 are respectively inserted and connected to each wavy opening 161 of the two fixed portions 16. The endotracheal tube 50 is inserted through the aperture 13 and abutted on the inner wall 141 of the bite wall 14. Then the pressing portion 354 is pressed so that the abutment surface 311 of the pushing component 30 is moved to the endotracheal tube 50. When the abutment surface 311 is abutted on the endotracheal tube 50, the two blocks 333 are respectively buckled on the two protruding portions 157, thereby fixing the endotracheal tube 50 on the inner wall 141 of the bite wall 14. As the endotracheal tube holder 1 of the present invention only requires pushing the pushing component 30 to achieve securing the endotracheal tube 50, the period of processing the endotracheal tube 50 is reduced, thereby reducing patients' discomfort.

Besides, the endotracheal tube holder 1 of the present invention still has another benefit by the following components. The first cushion segment 32 can cushion the reaction force generated by the endotracheal tube 50 pressed by the bite wall 14 for reducing the vibration of the endotracheal tube 50, so that the patient's discomfort can be reduced. Furthermore, the second cushion segment 34 also can absorb the vibration generated by moving the pushing component 30, thereby reducing the patient's discomfort.

As shown in Fig. 4, each wavy opening 161 of the two fixed portions 16 can be quickly fixed on the endotracheal tube holder 1 of the present invention for reducing chance of the fixing belt 40 sliding inside the wavy openings 161, so that the endotracheal tube holder 1 of the present invention avoids falling from the patient's head.

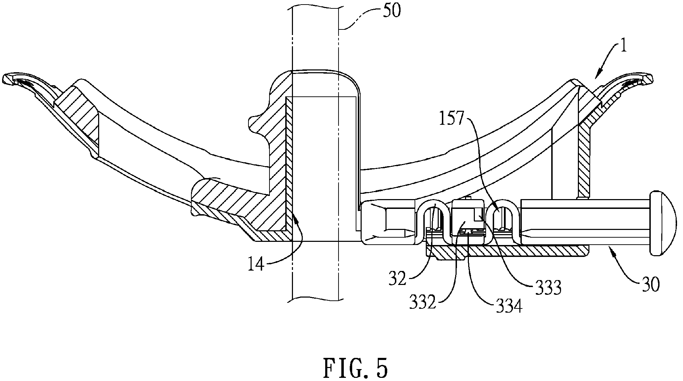

As shown in Fig. 5, when the pushing component 30 is moved toward endotracheal tube 50, the two blocks 333 are respectively pushed against the protrusions of the two protruding portions 157 so that the endotracheal tube holder 1 of the present invention vibrates. But, the two side grooves 334 allows the two middle side walls 332 to move inward to cushion the vibration generated by the two blocks 333 respectively pushing against the two protruding portions 157, thereby reducing the patient's discomfort.

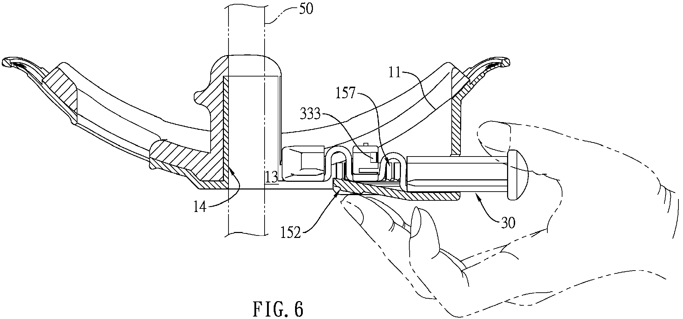

As shown in Fig. 6, when the endotracheal tube 50 is removed, the abutment plate 152 only needs to be pushed inward, and the two blocks 333 respectively depart from the two protruding portions 157, so that the pushing component 30 can be pulled away from the aperture 13, thereby removing the endotracheal tube 50.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. An endotracheal tube holder (1), **characterized in that** the endotracheal tube holder (1) comprises:
a holder body (10) comprising:
an inner surface (11);
an aperture (13) formed through the inner surface (11) and located at the middle of the holder body (10);
a bite wall (14) formed on and extending from the inner surface (11);
and
a receiving portion (15) comprising:
two side walls (151) parallel to each other and connected to the inner surface (11);
a receiving space (153) formed between the two side walls (151);
an opening (155) located at a side of the receiving portion (15) opposite to the bite wall (14);
the opening (155) and the receiving space (153) communicating with the aperture (13);
and
two protruding portions (157) respectively located at the two side walls (151);
and
two fixed portions (16) respectively located at two ends of the holder body (10) and connected to the inner surface (11);
a cushion (20) located at the inner surface (11) of the holder body (10) and the bite wall (14);
and
a pushing component (30) mounted through the opening (155), and the pushing component (30) comprising:
two middle side walls (332) parallel to each other;
and
two blocks (333) respectively located at the two middle side walls (332),
and
the two blocks (333) selectively buckled on the two protruding portions (157).

2. The endotracheal tube holder (1) as claimed in claim 1, wherein the receiving portion (15) further comprises:
an abutment plate (152) connected to a side of the two side walls (151) opposite to the inner surface (11);
two grooves (154) respectively located at the abutment plate (152) and extending to the aperture (13), and the two grooves (154) respectively parallel to the two side walls (151).

3. The endotracheal tube holder (1) as claimed in claim 2, wherein the pushing component (30) comprises:
a front segment (31) having an abutment surface (311) and two front side walls (312), two ends of the abutment surface (311) respectively connected to the two front side walls (312), and the two front side walls (312) parallel to each other;
a middle segment (33) comprising a bottom wall (331), the two middle side walls (332) and the two blocks (333); the bottom wall (331) located at an end of the front segment (31) that is opposite to the abutment surface (311), and the two middle side walls (332) respectively connected to two sides of the bottom wall (331); and
a pressing segment (35) located at an end of the bottom wall (331) opposite to the front segment (31).

4. The endotracheal tube holder (1) as claimed in claim 3, wherein the middle segment (33) further comprises two side grooves (334) respectively located at the connection site between the two middle side walls (332) and the bottom wall (331).

5. The endotracheal tube holder (1) as claimed in claim 3, wherein the pushing component (30) further comprises a first cushion segment (32) located between the front segment (31) and the middle segment (33).

6. The endotracheal tube holder (1) as claimed in claim 3, wherein the pushing component (30) further comprises a second cushion segment (34) located between the middle segment (33) and the pressing segment (35).

7. The endotracheal tube holder (1) as claimed in claim 3, wherein the receiving portion (15) further comprises at least one guiding groove (156) formed on and extending from the opening (155) connected to the abutment plate (152); wherein the front segment (31) further comprises at least one front guiding strip (313) located at one of the two front side walls (312), and the at least one front guiding strip (313) is slid into the at least one guiding groove (156).

8. The endotracheal tube holder (1) as claimed in claim 3, wherein the receiving portion (15) further comprises at least one guiding groove (156) formed on and extending from the opening (155) connected to the abutment plate (152); wherein the pressing segment (35) comprises:
a rear bottom wall (351), an end of the rear bottom wall (351) connected to the bottom wall (331);
two rear side walls (352) respectively connected to two sides of the rear bottom wall (351), and the two rear side walls (352) parallel to each other; and
at least one rear guiding strip (353) located at one of the two rear side walls (352), and the at least one rear guiding strip (353) slid into the at least one guiding groove (156).

9. The endotracheal tube holder (1) as claimed in any one of claims 1 to 8, wherein each one of the protruding portions (157) comprises multiple protrusions, each one of the protrusions has a first inclined plane (158) toward the opening (155); wherein each block (333) has a second inclined plane (335) toward the bite wall (14).

10. The endotracheal tube holder (1) as claimed in any one of claims 1 to 8, wherein each fixed portion (16) has a wavy opening (161).
